# EUROPEAN PATENT SPECIFICATION

(11) **EP 3 669 900 B1**
(45) Date of publication and mention of the grant of the patent: **16.03.2022**
(21) Application number: 18215701.6
(22) Date of filing: 21.12.2018
(51) Int. Cl.: A61L 27/36, A61L 27/54, C12N 15/113, C12N 15/867, A61P 27/00

(54) **GENETICALLY MANIPULATED CORNEA FOR TRANSPLANTATION**
GENETISCH MODIFIZIERTE HORNHAUT ZUR TRANSPLANTATION
CORNÉE GÉNÉTIQUEMENT MANIPULÉE POUR LA TRANSPLANTATION

(43) Date of publication of application: 24.06.2020
(73) Proprietor: Medizinische Hochschule Hannover, 30625 Hannover (DE)
(72) Inventor: BLASCZYK, Rainer, 30916 Isernhagen (DE); FERREIRA DE FIGUEIREDO, Constanca, 31535 Neustadt am Rübenberge (DE)
(74) Representative: Taruttis, Stefan Georg

(56) References cited:
- WO-A1-2016/057536
- US-A1- 2005 287 129

## Description

The present invention relates to an ex vivo genetically manipulated cornea for use in allogeneic transplantation, especially for use in transplantation to patients lacking the immune privilege of the eye, e.g. in patients who have developed adaptive or humoral immune response, e.g. who have previously received a cornea transplant, have neovascularization and/or an inflammation in the eye. Further, the invention relates to the ex vivo method of production of the genetically manipulated cornea for use in allogeneic transplantation, and to the use of nucleic acid constructs, e.g. viral vectors and viral particles, in the method of production.

### State of art

US 2005/0287129 A1 describes reducing the expression of MHC class I or beta-2-microglobulin proteins by transfection of cells to be transplanted with a vector containing interfering RNA after analysing the degree of HLA matching between a donor graft and a recipient, and graft rejection is reduced by selectively suppressing a mismatched HLA.

WO 2016/057536 A1 describes removing MHC class I antigen from a transplant by means of enzymatic cleavage or by genetic modification in order to reduce the expression of immunogenic HLA I or CIITA.

Figueiredo et al., Suppl. to Transplantation 1572 (2012) describes corneal transplants expressing shRNA directed against β2-microglobulin.

Figueiredo and Blasczyk, INTECH, p. 314 (2016) describe lentiviral genetic manipulation of allograft tissue to express shRNA directed against β2-microglobulin or against the α-chain of HLA-DR for decreasing immunogenicity of the tissue.

### Object of the invention

An object of the invention is to provide an alternative ex vivo genetically manipulated cornea for use in allogeneic transplantation and that preferably is less immunogenic, and an alternative ex vivo method of production of ex vivo genetically manipulating a cornea, wherein the method preferably produces a cornea which is less immunogenic.

### Description of the invention

The scope of this invention is defined by the claims. Any "embodiment" or "example" which is disclosed in the description but is not covered by the claims should be considered as presented for illustrative purposes only. The invention provides a cornea, which is ex vivo, e.g. isolated, for use in transplantation and a method for production of a cornea, which is performed on an initial ex vivo, e.g. isolated cornea, for use in transplantation according to the claims. Accordingly, the cornea can be for use in the treatment of cornea defects. The cornea is genetically manipulated in order to only elicit a reduced immune response in an allogeneic recipient, preferably in order to not elicit an immune response in an allogeneic recipient Specifically, the initial cornea is alive, e.g. an explanted cornea from an allogeneic donor, and kept under cell culture conditions, preferably in a serum-free cell-culture medium. This initial cornea is immunologically not compatible with the later recipient, e.g. the initial cornea is MHC-mismatched with the MHC of the later recipient. The initial cornea is isolated, e.g. maintained in organ culture medium. Optionally, the initial cornea is of non-human origin.

The cornea is genetically manipulated to contain a nucleic acid construct containing an expression cassette encoding inhibitory RNA sequences, e.g. siRNA, preferably short-hairpin RNA (shRNA), for use in human initial corneas is specific for the transcript of all of the alpha or beta-chain of HLA-DR, DQ and DP molecules, and optionally further encoding an inhibitory RNA (shRNA) specific for the transcript of the class II transactivator gene (CIITA), preferably further in combination with an expression cassette encoding an inhibitory RNA e.g. siRNA, preferably short-hairpin RNA (shRNA), specific for the transcript of the β2-microglobulin (β2m) gene. The cornea obtained by the process is for use as a transplant, e.g. as a replacement for a damaged cornea, in an immunologically non-compatible recipient, especially for use in a recipient whose MHC is not compatible to the MHC of the cornea initially used in the method for production.

The expression cassettes contain a promoter controlling the transcription of the encoded gene, preferably a constitutive promoter, e.g. the HI promoter (SEQ ID NO: 5) or the U6 promoter (SEQ ID NO: 13) or the elongation factor short (EFS) promoter (SEQ ID NO: 6) or the EF1alpha promoter (SEQ ID NO: 7).

For introduction of the nucleic acid construct into the cornea, the nucleic acid construct can be contained in a viral vector, e.g. in a lentiviral vector, preferably packaged in lentiviral particles.

An ex vivo cornea containing an expression cassette encoding siRNA specific for the transcript of the alpha-chain or of the beta-chain of HLA class II genes, i.e. specific for the transcript of all of the alpha-chain of HLA-DR, DQ and DP molecules and/or all of the beta-chain of HLA-DR, DQ and DP molecules, and optionally an expression cassette encoding siRNA specific for the transcript of the class II transactivator gene (CIITA), preferably also an expression cassette encoding siRNA specific for the transcript of β2m has been found to effect significantly less immunologic responses after transplantation, e.g. essentially no significant immunologic response after transplantation, even e.g. in recipients having neovascularizations in the eye, previous or present inflammation in the eye and/or previous contact with allogenic tissue, e.g. patients who have developed an immune response against allogenic HLA molecules, e.g. patients who had previously received an allogeneic transplant. The introduction of an expression cassette encoding siRNA specific for the transcript of the human alpha-chain or of the beta-chain of HLA class II gene for an initial human cornea, and optionally introduction of an expression cassette encoding siRNA specific for the transcript of the class II transactivator gene (CIITA) has been found to result in the reduction, respectively suppression of the translation of MHC II in the cornea, and introduction of an expression cassette encoding shRNA specific for the transcript of β2m has been found to result in the reduction, respectively suppression of the translation of MHC I in the cornea.

It was found that transplantation of a cornea expressing siRNA specific for the transcript of the class II transactivator gene (CIITA), preferably also expressing shRNA specific for the transcript of β2m, does not induce an immunogenic reaction by the recipient, e.g. no de novo formation of alloantibodies, and does not result in graft rejection in vivo. Preferably, the genetically manipulated cornea of the invention after transplantation does not elicit an immune response in the recipient.

An shRNA specific for the mouse β2m gene transcript (MM_shβ2m) can comprise or consist of SEQ ID NO: 1, corresponding to the nucleotide sequence of 51 nt (nucleotides) starting at nucleotide No. 209 of X01838 (European Nucleotide Archive), an shRNA specific for the mouse alpha-chain of the H2E gene transcript can comprise of consist of SEQ ID NO: 2, corresponding to the nucleotide sequence of 51 nt starting at nucleotide No. 324 of U13648.1 (European Nucleotide Archive).

An shRNA specific for the human β2m gene transcript (HS_shβ2m) can comprise or consist of SEQ ID NO: 3, corresponding to the nucleotide sequence of 47 nt (nucleotides) starting at nucleotide No. 244 of NM_004048.2 (NCBI databank), an shRNA specific for the human class II transactivator gene transcript (HS-shCIITA) can comprise of consist of SEQ ID NO: 4, corresponding to the nucleotide sequence of 51 nt starting at nucleotide No. 400 of NM_000246 at (NCBI databank).

The shRNA loop contained is TTCAAGAGA.

A preferred promoter for driving the transcription of the shRNA encoding sequences is the HI promoter, e.g. comprising or consisting of SEQ ID NO: 5.

The elongation factor short (EFS) promoter preferably comprises or consists of SEQ ID NO: 6, the EF1-alpha promoter preferably comprises or consists of SEQ ID NO: 7.

An exemplary lentiviral vector backbone has SEQ ID NO: 8, which contains a shRNA cloning site at nt 5312, and 5629.

For simultaneously targeting and inibiting HLA-DRA, HLA-DPA1 and HLA-DQA, shRNA of SEQ ID NO: 10 or of SEQ ID NO: 11 or a combination of these can be used.

For inhibiting CIITA, shRNA of SEQ ID NO: 12 (start nt 400, aa 90) can be used.

Here below embodiments and examples are disclosed where an expression cassette encoding siRNA specific for the transcript of the alpha-chain of the H2E gene is used. These embodiments and examples are to be considered as reference-embodiments and reference-examples not according to the claimed invention. According to the claimed invention an expression cassette encoding siRNA specific for the transcript for the alpha-chain and/or for the beta-chain of the MHC II gene for all of HLA-DR, DQ and DP molecules is used. In the process for producing the genetically manipulated cornea, an explanted live cornea is washed, e.g. in serum-free organ culture medium. A preferred organ culture medium is Human Endothelial SFM (Gibco) or Culture Medium II (Biochrom) supplemented with 10ng/ml FGF-2. The cornea is kept in serum-free organ culture medium and transfected with the nucleic acid construct containing the expression cassettes encoding siRNA specific for the transcript of the alpha-chain of the H2E gene and/or expressing siRNA specific for the transcript of the class II transactivator gene (CIITA), preferably also expressing siRNA specific for the transcript of β2m. Preferably, the nucleic acid construct is contained in a viral vector, e.g. in a lentiviral vector, packaged into lentiviral particles, and introduced into the medium. Following an incubation period, e.g. for 2h at 37 °C under cell culture conditions, the cornea is washed, e.g. in organ culture medium, and optionally incubated in fresh organ culture medium, e.g. for 48h at 37°C. Following this incubation, the cornea can be transplanted into a recipient.

### Reference-

### Example: Genetically manipulating mouse cornea in vitro for use as a transplant

Corneas from C3H/HeJ (MHC haplotype H-2^{k}) or from Balb/cJ (control, MHC matched) mice were explanted and transduced with lentiviral particles containing nucleic acid constructs encoding an expression cassette for shRNA specific for silencing mRNA for β2-microglobulin of SEQ ID NO: 1, and/or encoding an expression cassette for shRNA of SEQ ID NO: 9 (shRNA specific for the murine shCIITA, NM001302618.1 (start 992, aa304), GCTGACCTCCCGTGTAAATGA TTCAAGAGA TCATTTACACGGGAGGTCAGC) specific for silencing murine CIITA transcripts, and/or encoding shRNA of SEQ ID NO: 2 specific for silencing the murine alpha-chain of H2E. The expression cassettes were contained in a lentiviral vector according to SEQ ID NO: 8, which was packaged in lentiviral particles. For transduction, the corneas were incubated in organ culture medium (culture medium II, Biochrom) containing 8 µg/mL protamine sulfate for 2h at 37°C. Afterwards, the medium containing the viral was replaced with fresh organ culture medium and the corneas were incubated for 48h at 37°C to produce the genetically manipulated corneas. Then, the genetically manipulated corneas (MHC haplotype H-2^{d}) were transplanted into new C3H/HeJ (MHC haplotype H-2^{k}) mice which were completely mismatched for MHC.

The organ culture medium could be replaced by cell culture medium.

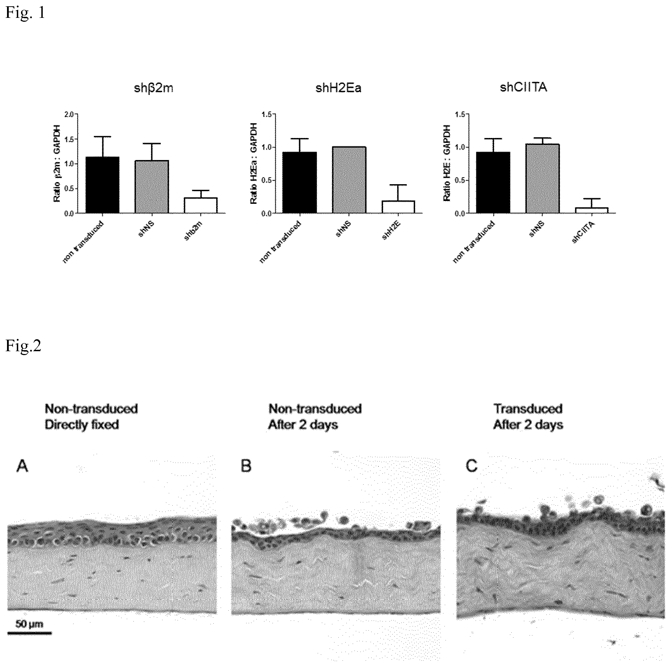

From the corneas that were genetically manipulated in vitro, resp. ex vivo, a section was taken and analysed by quantitative RT-PCR for transcripts of β2-microglobulin, of the alpha-chain of H2E, and of the CIITA. The results of the analyses for corneas that were transduced with a lentiviral nucleic acid construct containing an expression cassette for one of shRNA specific for silencing β2-microglobulin (shβ2m), shRNA specific for silencing the alpha-chain of H2E (shH2Ea) or shRNA specific for silencing CIITA (shCIITA) are depicted in Fig. 1, each normalized for the concentration of the mRNA for GAPDH. For comparison, non-transduced corneas and corneas transduced with an expression cassette encoding for the control shRNA shNS are shown. These results show that silencing of β2-microglobulin, of the alpha-chain of H2E, or of the CIITA was effective.

Fig. 2 shows histological analyses of the corneas in cross-sections in hematoxylin and eosin (HE) staining, in A of a freshly explanted mouse cornea, in B of a non-transduced cornea after two days of culture in organ culture medium at 37°C, and in C a cornea produced by the in vitro method of production for the genetic manipulation by transduction. These analyses show that the method of production essentially maintains the structure of the cornea.

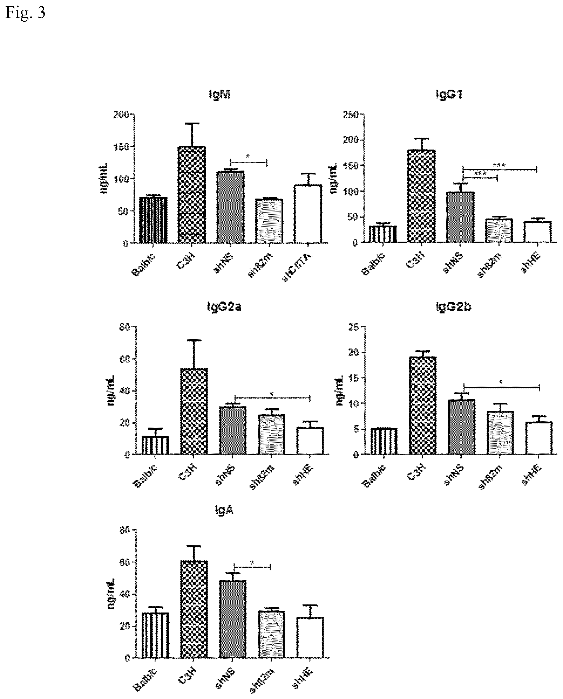

9 weeks after transplantation, mice were killed and the antibody levels in the sera as well as the tissue of the transplanted corneas were analysed. The results, which are depicted in Fig. 3, show a significant decrease in the levels of IgA, IgG1 and IgM antibodies in animals transplanted with a MHC class I silenced corneas. Interestingly, significant decreases in the levels of IgG1, IgG2a and IgG2b were detectable in the sera of animals transplanted with a MHC class II silenced cornea. Also, lower levels of IgA and IgM were detectable in these animals in comparison to those receiving non-modified corneas or expressing a non-specific shRNA. These data shows that both MHC class I and class II corneas are significantly less immunogenic and trigger a much weaker antibody mediated alloimmune response in comparison to fully MHC expressing corneas.

Analysis for T-cell infiltration showed that significantly (p<0.05) lower rates of T-cells were observed in MHC-mismatched corneas expressing one of shRNA specific for silencing β2-microglobulin, shRNA specific for silencing CIITA or shRNA specific for silencing the alpha-chain of H2E.

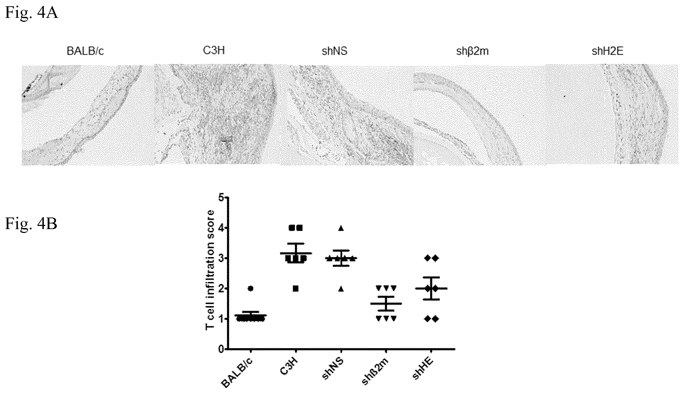

The results are shown in Fig. 4B, which were obtained from the tissue analyses of Fig. 4A using Luminex technology and classifying infiltration by T-cells to scores 1 (little or no T-cell infiltration) to 4 (massive T-cell infiltration).

Fig. 4 shows histological analyses of the corneas that were transplanted after 9 weeks in the recipient, in cross-sections with antibody staining for CD3 T-cells, and HE staining. For comparison, non-manipulated cornea from a Balb/c mouse that was transplanted into a Balb/c recipient mouse is shown, and a non-manipulated cornea transplanted and then retrieved from a C3H mouse, a C3H cornea expressing shNS, and the cornea that was genetically manipulated to express shRNA specific for silencing β2-microglobulin (shβ2m) and the cornea that was genetically manipulated to express shRNA specific for silencing the alpha-chain of H2E (shH2E). These results show that corneas silenced for β2-microglobulin showed a significantly decrease in immune cell infiltration and a better tissue structure. MHC class II silenced corneas showed a tendency for decreased T-cell infiltration.

## Claims

1. Cornea, ex vivo, for use in transplantation to a recipient that is immunologically non-compatible to an initial live isolated cornea, **characterized in that** the initial live cornea is ex vivo genetically manipulated to contain an expression cassette expressing at least one inhibitory RNA specific for the alpha-chain and/or for the beta-chain of the MHC II gene for all of HLA-DR, DQ and DP molecules.

2. Cornea for use in transplantation according to claim 1, **characterized in that** the cornea is genetically manipulated to additionally contain an expression cassette expressing an inhibitory RNA specific for the β2-microglobulin mRNA.

3. Cornea for use in transplantation according to one of the preceding claims, **characterized in that** the inhibitory RNA is specific for the beta-chain of the HLA-DR, DQ and DP molecules.

4. Cornea for use in transplantation according to one of the preceding claims, **characterized in that** the initial live cornea is genetically manipulated to contain an expression cassette expressing an inhibitory RNA specific for the class II transactivator gene (CIITA) .

5. Cornea for use in transplantation according to one of the preceding claims, **characterized in that** the initial cornea is of human origin.

6. Cornea for use in transplantation according to one of the preceding claims, wherein the cornea is for use in the treatment of a defective cornea.

7. Cornea for use in transplantation according to one of the preceding claims, **characterized in that** the recipient has neovascularizations in the eye, previous or present inflammation in the eye and/or previous contact with allogenic tissue, has developed an immune response against allogenic HLA molecules, or has previously received an allogeneic transplant.

8. Method for producing a cornea by ex-vivo genetically manipulating an initial isolated live cornea to contain an expression cassette expressing an inhibitory RNA specific for the alpha-chain and/or beta-chain of MHC II gene,
wherein the inhibitory RNA is specific for the alpha-chain and/or beta-chain for all of the HLA-DR, DQ and DP molecules.

9. Method according to claim 8, **characterized in that** the cornea is genetically manipulated to contain an expression cassette expressing an inhibitory RNA specific for the class II transactivator gene (CIITA).

10. Method according to claim 8 or 9, **characterized in that** the cornea is genetically manipulated to additionally contain an expression cassette expressing an inhibitory RNA specific for the β2-microglobulin mRNA.

11. Method according to one of claims 8 to 10, **characterized in that** the initial cornea is maintained in serum-free organ culture medium, at least one nucleic acid construct containing the expression cassettes packaged in viral particles is added to the medium, the cornea is kept in the medium for an incubation period, and the medium is replaced by fresh serum-free medium for an incubation period sufficient for transcription of the expression cassettes.

12. Method according to one of claims 8 to 11, **characterized in that** the at least one nucleic acid construct which is added to the medium is a lentiviral vector which is packaged in lentiviral particles.

## Patentansprüche

1. Hornhaut, ex vivo, zur Verwendung bei der Transplantation an einen Empfänger, der immunologisch nicht kompatibel mit einer ursprünglichen lebenden isolierten Hornhaut ist, **dadurch gekennzeichnet, dass** die ursprüngliche lebende Hornhaut ex vivo genetisch manipuliert ist, um eine Expressionskassette zu enthalten, die mindestens eine für die alpha-Kette und/oder für die beta-Kette des MHC-II-Gens aller HLA-DR-, DQ- und DP-Moleküle spezifische inhibitorische RNA exprimiert.

2. Hornhaut zur Verwendung bei der Transplantation nach Anspruch 1, **dadurch gekennzeichnet, dass** die Hornhaut genetisch manipuliert ist, um zusätzlich eine Expressionskassette zu enthalten, die eine für die β2-Mikroglobulin-mRNA spezifische inhibitorische RNA exprimiert.

3. Hornhaut zur Verwendung bei der Transplantation nach einem der voranstehenden Ansprüche, **dadurch gekennzeichnet, dass** die inhibitorische RNA für die beta-Kette der HLA-DR-, DQ- und DP-Moleküle spezifisch ist.

4. Hornhaut zur Verwendung bei der Transplantation nach einem der voranstehenden Ansprüche, **dadurch gekennzeichnet, dass** die ursprüngliche lebende Hornhaut genetisch manipuliert ist, um eine Expressionskassette zu enthalten, die eine für die mRNA des Klasse-II-Transaktivatorgens (CIITA) spezifische inhibitorische RNA exprimiert

5. Hornhaut zur Verwendung bei der Transplantation nach einem der voranstehenden Ansprüche, **dadurch gekennzeichnet, dass** die ursprüngliche lebende Hornhaut menschlichen Ursprungs ist.

6. Hornhaut zur Verwendung bei der Transplantation nach einem der voranstehenden Ansprüche, wobei die Hornhaut zur Verwendung bei der Behandlung einer defekten Hornhaut ist.

7. Hornhaut zur Verwendung bei der Transplantation nach einem der voranstehenden Ansprüche, **dadurch gekennzeichnet, dass** der Empfänger Neovaskularisationen im Auge, vorherige oder gegenwärtige Entzündungen im Auge und/oder Kontakt mit allogenem Gewebe hat, eine Immunantwort gegen allogene HLA-Moleküle entwickelt hat oder vorher ein allogenes Transplantat erhalten hat.

8. Verfahren zur Herstellung einer Hornhaut durch ex vivo genetisches Manipulieren einer ursprünglichen isolierten lebenden Hornhaut, dass sie eine Expressionskassette enthält, die eine inhibitorische RNA exprimiert, die für die alpha-Kette und/oder beta-Kette des MHC-II-Gens spezifisch ist, wobei die inhibitorische RNA für die alpha-Kette und/oder beta-Kette aller HLA-DR-, DQ- und DP-Moleküle spezifisch ist.

9. Verfahren nasch Anspruch 8, **dadurch gekennzeichnet, dass** die Hornhaut genetisch manipuliert wird, dass sie eine Expressionskassette enthält, die eine inhibitorische RNA exprimiert, die spezifisch für das Klasse-II-Transaktivator-Gen (CIITA) ist.

10. Verfahren nach Anspruch 8 oder 9, **dadurch gekennzeichnet, dass** die Hornhaut genetisch manipuliert wird, dass sie zusätzlich eine Expressionskassette enthält, die eine für die β2-Mikroglobulin-mRNA spezifische inhibitorische RNA exprimiert.

11. Verfahren nach einem der Ansprüche 8 bis 10, **dadurch gekennzeichnet, dass** die ursprüngliche Hornhaut in serumfreiem Organkulturmedium gehalten wird, dem Medium mindestens ein Nukleinsäurekonstrukt zugesetzt wird, das die in Viruspartikel verpackten Expressionskassetten enthält, die Hornhaut im Medium für eine Inkubationszeit gehalten wird, und das Medium durch frisches serumfreies Medium für eine Inkubationszeit ersetzt wird, die für die Transkription der Expressionskassetten ausreichend ist.

12. Verfahren nach einem der Ansprüche 8 bis 11, **dadurch gekennzeichnet, dass** das mindestens eine dem Medium zugesetzte Nukleinsäurekonstrukt ein lentiviraler Vektor ist, der in lentivirale Partikel verpackt ist.

## Revendications

1. Cornée, ex vivo, destinée à être utilisée dans la transplantation à un receveur qui est immunologiquement non compatible avec une cornée vivante isolée initiale, **caractérisée en ce que** la cornée vivante initiale est ex vivo génétiquement manipulée pour contenir une cassette d'expression exprimant au moins un ARN inhibiteur spécifique pour la chaîne alpha et/ou pour la chaîne bêta du gène MHC II de l'ensemble des molécules HLA-DR, -DQ et -DP.

2. Cornée destinée à être utilisée en transplantation selon la revendication 1, **caractérisée en ce que** la cornée est manipulée génétiquement pour contenir en outre une cassette d'expression exprimant un ARN inhibiteur spécifique de l'ARNm de la β2-microglobuline.

3. Cornée destinée à être utilisée en transplantation selon l'une des revendications précédentes, **caractérisée en ce que** l'ARN inhibiteur est spécifique de la chaîne bêta des molécules HLA-DR, -DQ et -DP.

4. Cornée destinée à être utilisée en transplantation selon l'une des revendications précédentes, **caractérisée en ce que** la cornée vivante initiale est manipulée génétiquement pour contenir une cassette d'expression exprimant un ARN inhibiteur spécifique du gène transactivateur de classe II (CIITA).

5. Cornée destinée à être utilisée en transplantation selon l'une des revendications précédentes, **caractérisée en ce que** la cornée initiale est d'origine humaine.

6. Cornée destinée à être utilisée en transplantation selon l'une des revendications précédentes, **caractérisée en ce que** la cornée est utilisée dans le traitement d'une cornée défectueuse.

7. Cornée destinée à être utilisée en transplantation selon l'une des revendications précédentes, **caractérisée en ce que** le receveur présentant des néovascularisations dans l'œil, une inflammation antérieure ou actuelle de l'œil et/ou un contact antérieur avec un tissu allogénique, a développé une réponse immunitaire contre les molécules HLA allogéniques, ou a reçu antérieurement une greffe allogénique.

8. Procédé de production d'une cornée par ex vivo manipulation génétique d'une cornée vivante isolée initiale pour contenir une cassette d'expression exprimant un ARN inhibiteur spécifique de la chaîne alpha et/ou de la chaîne bêta du gène MHC II de la totalité des molécules HLA-DR, DQ et DP.

9. Procédé selon la revendication 8, **caractérisé en ce que** la cornée est manipulée génétiquement pour contenir l'ARN inhibiteur spécifique du gène transactivateur de classe II (CIITA).

10. Procédé selon la revendication 8 ou 9, **caractérisé en ce que** la cornée est manipulée génétiquement pour contenir en outre une cassette d'expression exprimant un ARN inhibiteur spécifique de l'ARNm de la β2-microglobuline.

11. Procédé selon l'une des revendications 8 à 10, **caractérisé en ce que** la cornée initiale est maintenue dans un medium de culture d'organes sans sérum, au moins une construction d'acide nucléique contenant les cassettes d'expression conditionnées dans des particules virales est ajoutée au médium, la cornée est maintenue dans le medium pendant une période d'incubation, et le medium est remplacé par du medium frais sans sérum pendant une période d'incubation suffisante pour la transcription des cassettes d'expression.

12. Procédé selon l'une des revendications 8 à 11, **caractérisé en ce que** la au moins une construction d'acide nucléique qui est ajoutée au milieu est un vecteur lentiviral qui est conditionné dans des particules lentivirales.


### SEQUENCE LISTING

<110> Medizinische Hochschule Hannover
<120> Genetically manipulated cornea for transplantation
<130> M1078EP
<160> 13
<170> BiSSAP 1.3.6
<210> 1
   <211> 51
   <212> DNA
   <213> Artificial Sequence
<220>
   <223> shRNA specific for mouse beta-2-microglobulin
<400> 1
   gcctcacatt gaaatccaaa tttcaagaga tatagaaaga ccagtccttg c 51
<210> 2
   <211> 51
   <212> DNA
   <213> Artificial Sequence
<220>
   <223> shRNA specific for mouse alpha-chain of H2E
<400> 2
   gcgttccaac aacactccag attcaagaga tctggagtgt tgttggaacg c 51
<210> 3
   <211> 47
   <212> DNA
   <213> Artificial Sequence
<220>
   <223> shRNA specific for human beta-2-microglobulin
<400> 3
   gaatggagag agaattgaat tcaagagatt caattctctc tccattc 47
<210> 4
   <211> 51
   <212> DNA
   <213> Artificial Sequence
<220>
   <223> shRNA specific for human CIITA
<400> 4
   aagagaccag ggaggcttat gttcaagaga cataagcctc cctggtctct t 51
<210> 5
   <211> 220
   <212> DNA
   <213> Artificial Sequence
<220>
   <223> H1 promoter for transcription of shRNA
<400> 5
   aattcgaacg ctgacgtcat caacccgctc caaggaatcg cgggcccagt gtcactaggc 60
   gggaacaccc agcgcgcgtg cgccctggca ggaagatggc tgtgagggac aggggagtgg 120
   cgccctgcaa tatttgcatg tcgctatgtg ttctgggaaa tcaccataaa cgtgaaatgt 180
   ctttggattt gggaatctta taagttctgt atgagaccac 220
<210> 6
   <211> 245
   <212> DNA
   <213> Artificial Sequence
<220>
   <223> EFS
<400> 6
   tggctccggt gcccgtcagt gggcagagcg cacatcgccc acagtccccg agaagttggg 60
   gggaggggtc ggcaattgaa ccggtgccta gagaaggtgg cgcggggtaa actgggaaag 120
   tgatgtcgtg tactggctcc gcctttttcc cgagggtggg ggagaaccgt atataagtgc 180
   agtagtcgcc gtgaacgttc tttttcgcaa cgggtttgcc gccagaacac aggtgtcgtg 240
   acgcg 245
<210> 7
   <211> 1080
   <212> DNA
   <213> Artificial Sequence
<220>
   <223> EF1 alpha
<400> 7
   catttcaggt gtcgtgagga atttcgacat ttaaatttaa ttaatctcga cggtatcggt 60
   taacttttaa aagaaaaggg gggattgggg ggtacagtgc aggggaaaga atagtagaca 120
   taatagcaac agacatacaa actaaagaat tacaaaaaca aattacaaaa attcaaaatt 180
   ttatcgatca cgagactagc ctcgaggttt aaactacggg atcttcgaag gcctaagctt 240
   acgcgcgcgt cctagcgcta ccggtcgcca ccatggtgag caagggcgag gagctgttca 300
   ccggggtggt gcccatcctg gtcgagctgg acggcgacgt aaacggccac aagttcagcg 360
   tgtccggcga gggcgagggc gatgccacct acggcaagct gaccctgaag ttcatctgca     420
   ccaccggcaa gctgcccgtg ccctggccca ccctcgtgac caccctgacc tacggcgtgc     480
   agtgcttcag ccgctacccc gaccacatga agcagcacga cttcttcaag tccgccatgc     540
   ccgaaggcta cgtccaggag cgcaccatct tcttcaagga cgacggcaac tacaagaccc     600
   gcgccgaggt gaagttcgag ggcgacaccc tggtgaaccg catcgagctg aagggcatcg     660
   acttcaagga ggacggcaac atcctggggc acaagctgga gtacaactac aacagccaca     720
   acgtctatat catggccgac aagcagaaga acggcatcaa ggtgaacttc aagatccgcc     780
   acaacatcga ggacggcagc gtgcagctcg ccgaccacta ccagcagaac acccccatcg     840
   gcgacggccc cgtgctgctg cccgacaacc actacctgag cacccagtcc gccctgagca     900
   aagaccccaa cgagaagcgc gatcacatgg tcctgctgga gttcgtgacc gccgccggga     960
   tcactctcgg catggacgag ctgtacaagt ccggactcag atctcgacta gctagtagct    1020
   agctagctag tcgagctcaa gcttcgggga ctagtcatat gataatcaac ctctggatta    1080
<210> 8
   <211> 11085
   <212> DNA
   <213> Artificial Sequence
<220>
   <223> empty vector backbone
<400> 8
   ttggaagggc taattcactc ccaaagaaga caagatatcc ttgatctgtg gatctaccac     60
   acacaaggct acttccctga ttagcagaac tacacaccag ggccaggggt cagatatcca    120
   ctgacctttg gatggtgcta caagctagta ccagttgagc cagataaggt agaagaggcc    180
   aataaaggag agaacaccag cttgttacac cctgtgagcc tgcatgggat ggatgacccg    240
   gagagagaag tgttagagtg gaggtttgac agccgcctag catttcatca cgtggcccga    300
   gagctgcatc cggagtactt caagaactgc tgatatcgag cttgctacaa gggactttcc    360
   gctggggact ttccagggag gcgtggcctg ggcgggactg gggagtggcg agccctcaga    420
   tcctgcatat aagcagctgc tttttgcctg tactgggtct ctctggttag accagatctg    480
   agcctgggag ctctctggct aactagggaa cccactgctt aagcctcaat aaagcttgcc    540
   ttgagtgctt caagtagtgt gtgcccgtct gttgtgtgac tctggtaact agagatccct    600
   cagacccttt tagtcagtgt ggaaaatctc tagcagtggc gcccgaacag ggacttgaaa    660
   gcgaaaggga aaccagagga gctctctcga cgcaggactc ggcttgctga agcgcgcacg    720
   gcaagaggcg aggggcggcg actggtgagt acgccaaaaa ttttgactag cggaggctag    780
   aaggagagag atgggtgcga gagcgtcagt attaagcggg ggagaattag atcgcgatgg    840
   gaaaaaattc ggttaaggcc agggggaaag aaaaaatata aattaaaaca tatagtatgg 900
   gcaagcaggg agctagaacg attcgcagtt aatcctggcc tgttagaaac atcagaaggc 960
   tgtagacaaa tactgggaca gctacaacca tcccttcaga caggatcaga agaacttaga 1020
   tcattatata atacagtagc aaccctctat tgtgtgcatc aaaggataga gataaaagac 1080
   accaaggaag ctttagacaa gatagaggaa gagcaaaaca aaagtaagac caccgcacag 1140
   caagcggccg ctgatcttca gacctggagg aggagatatg agggacaatt ggagaagtga 1200
   attatataaa tataaagtag taaaaattga accattagga gtagcaccca ccaaggcaaa 1260
   gagaagagtg gtgcagagag aaaaaagagc agtgggaata ggagctttgt tccttgggtt 1320
   cttgggagca gcaggaagca ctatgggcgc agcgtcaatg acgctgacgg tacaggccag 1380
   acaattattg tctggtatag tgcagcagca gaacaatttg ctgagggcta ttgaggcgca 1440
   acagcatctg ttgcaactca cagtctgggg catcaagcag ctccaggcaa gaatcctggc 1500
   tgtggaaaga tacctaaagg atcaacagct cctggggatt tggggttgct ctggaaaact 1560
   catttgcacc actgctgtgc cttggaatgc tagttggagt aataaatctc tggaacagat 1620
   ttggaatcac acgacctgga tggagtggga cagagaaatt aacaattaca caagcttaat 1680
   acactcctta attgaagaat cgcaaaacca gcaagaaaag aatgaacaag aattattgga 1740
   attagataaa tgggcaagtt tgtggaattg gtttaacata acaaattggc tgtggtatat 1800
   aaaattattc ataatgatag taggaggctt ggtaggttta agaatagttt ttgctgtact 1860
   ttctatagtg aatagagtta ggcagggata ttcaccatta tcgtttcaga cccacctccc 1920
   aaccccgagg ggacccgaca ggcccgaagg aatagaagaa gaaggtggag agagagacag 1980
   agacagatcc attcgattag tgaacggatc tcgacggtat cgcgatgtcg acgataagct 2040
   ttgcaaagat ggataaagtt ttaaacagag aggaatcttt gcagctaatg gaccttctag 2100
   gtcttgaaag gagtgggaat tggctccggt gcccgtcagt gggcagagcg cacatcgccc 2160
   acagtccccg agaagttggg gggaggggtc ggcaattgaa ccggtgccta gagaaggtgg 2220
   cgcggggtaa actgggaaag tgatgtcgtg tactggctcc gcctttttcc cgagggtggg 2280
   ggagaaccgt atataagtgc agtagtcgcc gtgaacgttc tttttcgcaa cgggtttgcc 2340
   gccagaacac aggtaagtgc cgtgtgtggt tcccgcgggc ctggcctctt tacgggttat 2400
   ggcccttgcg tgccttgaat tacttccact ggctgcagta cgtgattctt gatcccgagc 2460
   ttcgggttgg aagtgggtgg gagagttcga ggccttgcgc ttaaggagcc ccttcgcctc 2520
   gtgcttgagt tgaggcctgg cctgggcgct ggggccgccg cgtgcgaatc tggtggcacc 2580
   ttcgcgcctg tctcgctgct ttcgataagt ctctagccat ttaaaatttt tgatgacctg 2640
   ctgcgacgct ttttttctgg caagatagtc ttgtaaatgc gggccaagat ctgcacactg 2700
   gtatttcggt ttttggggcc gcgggcggcg acggggcccg tgcgtcccag cgcacatgtt 2760
   cggcgaggcg gggcctgcga gcgcggccac cgagaatcgg acgggggtag tctcaagctg 2820
   gccggcctgc tctggtgcct ggcctcgcgc cgccgtgtat cgccccgccc tgggcggcaa 2880
   ggctggcccg gtcggcacca gttgcgtgag cggaaagatg gccgcttccc ggccctgctg 2940
   cagggagctc aaaatggagg acgcggcgct cgggagagcg ggcgggtgag tcacccacac 3000
   aaaggaaaag ggcctttccg tcctcagccg tcgcttcatg tgactccacg gagtaccggg 3060
   cgccgtccag gcacctcgat tagttctcga gcttttggag tacgtcgtct ttaggttggg 3120
   gggaggggtt ttatgcgatg gagtttcccc acactgagtg ggtggagact gaagttaggc 3180
   cagcttggca cttgatgtaa ttctccttgg aatttgccct ttttgagttt ggatcttggt 3240
   tcattctcaa gcctcagaca gtggttcaaa gtttttttct tccatttcag gtgtcgtgag 3300
   gaatttcgac atttaaattt aattaatctc gacggtatcg gttaactttt aaaagaaaag 3360
   gggggattgg ggggtacagt gcaggggaaa gaatagtaga cataatagca acagacatac 3420
   aaactaaaga attacaaaaa caaattacaa aaattcaaaa ttttccgatc acgagactag 3480
   cctcgaggtt taaactacgg gatcttcgaa ggcctaagct tacgcgcgcg tcctagcgct 3540
   accggtcgcc accatggtga gcaagggcga ggagctgttc accggggtgg tgcccatcct 3600
   ggtcgagctg gacggcgacg taaacggcca caagttcagc gtgtccggcg agggcgaggg 3660
   cgatgccacc tacggcaagc tgaccctgaa gttcatctgc accaccggca agctgcccgt 3720
   gccctggccc accctcgtga ccaccctgac ctacggcgtg cagtgcttca gccgctaccc 3780
   cgaccacatg aagcagcacg acttcttcaa gtccgccatg cccgaaggct acgtccagga 3840
   gcgcaccatc ttcttcaagg acgacggcaa ctacaagacc cgcgccgagg tgaagttcga 3900
   gggcgacacc ctggtgaacc gcatcgagct gaagggcatc gacttcaagg aggacggcaa 3960
   catcctgggg cacaagctgg agtacaacta caacagccac aacgtctata tcatggccga 4020
   caagcagaag aacggcatca aggtgaactt caagatccgc cacaacatcg aggacggcag 4080
   cgtgcagctc gccgaccact accagcagaa cacccccatc ggcgacggcc ccgtgctgct 4140
   gcccgacaac cactacctga gcacccagtc cgccctgagc aaagacccca acgagaagcg 4200
   cgatcacatg gtcctgctgg agttcgtgac cgccgccggg atcactctcg gcatggacga 4260
   gctgtacaag tccggactca gatctcgata gctagtagct agctagctag tcgagctcaa 4320
   cttcggggac tagtcatatg ataatcaacc tctggattac aaaatttgtg aaagattgac 4380
   tggtattctt aactatgttg ctccttttac gctatgtgga tacgctgctt taatgccttt 4440
   gtatcatgct attgcttccc gtatggcttt cattttctcc tccttgtata aatcctggtt 4500
   gctgtctctt tatgaggagt tgtggcccgt tgtcaggcaa cgtggcgtgg tgtgcactgt 4560
   gtttgctgac gcaaccccca ctggttgggg cattgccacc acctgtcagc tcctttccgg 4620
   gactttcgct ttccccctcc ctattgccac ggcggaactc atcgccgcct gccttgcccg 4680
   ctgctggaca ggggctcggc tgttgggcac tgacaattcc gtggtgttgt cggggaagct 4740
   gacgtccttt ccatggctgc tcgcctgtgt tgccacctgg attctgcgcg ggacgtcctt 4800
   ctgctacgtc ccttcggccc tcaatccagc ggaccttcct tcccgcggcc tgctgccggc 4860
   tctgcggcct cttccgcgtc ttcgccttcg ccctcagacg agtcggatct ccctttgggc 4920
   cgcctccccg catcggtacg tatggccagg tacctttaag accaatgact tacaaggcag 4980
   ctgtagatct tagccacttt ttaaaagaaa aggggggact ggaagggcta attcactccc 5040
   aacgaagaca agatgggatc aattcaccat gctagtggat ccccctcgag tttaccactc 5100
   cctatcagtg atagagaaaa gtgaaagtcg agtttaccac tccctatcag tgatagagaa 5160
   aagtgaaagt cgagtttacc actccctatc agtgatagag aaaagtgaaa gtcgagttta 5220
   ccactcccta tcagtgatag agaaaagtga aagtcgagtt taccactccc tatcagtgat 5280
   agagaaaagt gaaagtcgag tttaccactc cctatcagtg atagagaaaa gtgaaagtcg 5340
   agtttaccac tccctatcag tgatagagaa aagtgaaagt cggggctgca ggaattcgaa 5400
   cgctgacgtc atcaacccgc tccaaggaat cgcgggccca gtgtcactag gcgggaacac 5460
   ccagcgcgcg tgcgccctgg caggaagatg gctgtgaggg acaggggagt ggcgccctgc 5520
   aatatttgca tgtcgctatg tgttctggga aatcaccata aacgtgaaat gtctttggat 5580
   ttgggaatct tataagttct gtatgagacc acgcgtctcc aagcttatcg ataccgtcgc 5640
   atgggaataa cttcgtatag catacattat acgaagttat gctgcttttt gcttgtactg 5700
   ggtctctctg gttagaccag atctgagcct gggagctctc tggctaacta gggaacccac 5760
   tgcttaagcc tcaataaagc ttgccttgag tgcttcaagt agtgtgtgcc cgtctgttgt 5820
   gtgactctgg taactagaga tccctcagac ccttttagtc agtgtggaaa atctctagca 5880
   gcatctagaa ttaattccgt gtattctata gtgtcaccta aatcgtatgt gtatgataca 5940
   taaggttatg tattaattgt agccgcgttc taacgacaat atgtacaagc ctaattgtgt 6000
   agcatctggc ttactgaagc agaccctatc atctctctcg taaactgccg tcagagtcgg 6060
   tttggttgga cgaaccttct gagtttctgg taacgccgtc ccgcacccgg aaatggtcag 6120
   cgaaccaatc agcagggtca tcgctagcca gatcctctac gccggacgca tcgtggccgg 6180
   catcaccggc gccacaggtg cggttgctgg cgcctatatc gccgacatca ccgatgggga 6240
   agatcgggct cgccacttcg ggctcatgag cgcttgtttc ggcgtgggta tggtggcagg 6300
   ccccgtggcc gggggactgt tgggcgccat ctccttgcat gcaccattcc ttgcggcggc 6360
   ggtgctcaac ggcctcaacc tactactggg ctgcttccta atgcaggagt cgcataaggg 6420
   agagcgtcga atggtgcact ctcagtacaa tctgctctga tgccgcatag ttaagccagc 6480
   cccgacaccc gccaacaccc gctgacgcgc cctgacgggc ttgtctgctc ccggcatccg 6540
   cttacagaca agctgtgacc gtctccggga gctgcatgtg tcagaggttt tcaccgtcat 6600
   caccgaaacg cgcgagacga aagggcctcg tgatacgcct atttttatag gttaatgtca 6660
   tgataataat ggtttcttag acgtcaggtg gcacttttcg gggaaatgtg cgcggaaccc 6720
   ctatttgttt atttttctaa atacattcaa atatgtatcc gctcatgaga caataaccct 6780
   gataaatgct tcaataatat tgaaaaagga agagtatgag tattcaacat ttccgtgtcg 6840
   cccttattcc cttttttgcg gcattttgcc ttcctgtttt tgctcaccca gaaacgctgg 6900
   tgaaagtaaa agatgctgaa gatcagttgg gtgcacgagt gggttacatc gaactggatc 6960
   tcaacagcgg taagatcctt gagagttttc gccccgaaga acgttttcca atgatgagca 7020
   cttttaaagt tctgctatgt ggcgcggtat tatcccgtat tgacgccggg caagagcaac 7080
   tcggtcgccg catacactat tctcagaatg acttggttga gtactcacca gtcacagaaa 7140
   agcatcttac ggatggcatg acagtaagag aattatgcag tgctgccata accatgagtg 7200
   ataacactgc ggccaactta cttctgacaa cgatcggagg accgaaggag ctaaccgctt 7260
   ttttgcacaa catgggggat catgtaactc gccttgatcg ttgggaaccg gagctgaatg 7320
   aagccatacc aaacgacgag cgtgacacca cgatgcctgt agcaatggca acaacgttgc 7380
   gcaaactatt aactggcgaa ctacttactc tagcttcccg gcaacaatta atagactgga 7440
   tggaggcgga taaagttgca ggaccacttc tgcgctcggc ccttccggct ggctggttta 7500
   ttgctgataa atctggagcc ggtgagcgtg ggtctcgcgg tatcattgca gcactggggc 7560
   cagatggtaa gccctcccgt atcgtagtta tctacacgac ggggagtcag gcaactatgg 7620
   atgaacgaaa tagacagatc gctgagatag gtgcctcact gattaagcat tggtaactgt 7680
   cagaccaagt ttactcatat atactttaga ttgatttaaa acttcatttt taatttaaaa 7740
   ggatctaggt gaagatcctt tttgataatc tcatgaccaa aatcccttaa cgtgagtttt 7800
   cgttccactg agcgtcagac cccgtagaaa agatcaaagg atcttcttga gatccttttt 7860
   ttctgcgcgt aatctgctgc ttgcaaacaa aaaaaccacc gctaccagcg gtggtttgtt 7920
   tgccggatca agagctacca actctttttc cgaaggtaac tggcttcagc agagcgcaga 7980
   taccaaatac tgtccttcta gtgtagccgt agttaggcca ccacttcaag aactctgtag 8040
   caccgcctac atacctcgct ctgctaatcc tgttaccagt ggctgctgcc agtggcgata 8100
   agtcgtgtct taccgggttg gactcaagac gatagttacc ggataaggcg cagcggtcgg 8160
   gctgaacggg gggttcgtgc acacagccca gcttggagcg aacgacctac accgaactga 8220
   gatacctaca gcgtgagcat tgagaaagcg ccacgcttcc cgaagggaga aaggcggaca 8280
   ggtatccggt aagcggcagg gtcggaacag gagagcgcac gagggagctt ccagggggaa 8340
   acgcctggta tctttatagt cctgtcgggt ttcgccacct ctgacttgag cgtcgatttt 8400
   tgtgatgctc gtcagggggg cggagcctat ggaaaaacgc cagcaacgcg gcctttttac 8460
   ggttcctggc cttttgctgg ccttttgctc acatgttctt tcctgcgtta tcccctgatt 8520
   ctgtggataa ccgtattacc gcctttgagt gagctgatac cgctcgccgc agccgaacga 8580
   ccgagcgcag cgagtcagtg agcgaggaag cggaagagcg cccaatacgc aaaccgcctc 8640
   tccccgcgcg ttggccgatt cattaatgca gctgtggaat gtgtgtcagt tagggtgtgg 8700
   aaagtcccca ggctccccag caggcagaag tatgcaaagc atgcatctca attagtcagc 8760
   aaccaggtgt ggaaagtccc caggctcccc agcaggcaga agtatgcaaa gcatgcatct 8820
   caattagtca gcaaccatag tcccgcccct aactccgccc atcccgcccc taactccgcc 8880
   cagttccgcc cattctccgc cccatggctg actaattttt tttatttatg cagaggccga 8940
   ggccgcctcg gcctctgagc tattccagaa gtagtgagga ggcttttttg gaggcctagg 9000
   cttttgcaaa aagcttggac acaagacagg cttgcgagat atgtttgaga ataccacttt 9060
   atcccgcgtc agggagaggc agtgcgtaaa aagacgcgga ctcatgtgaa atactggttt 9120
   ttagtgcgcc agatctctat aatctcgcgc aacctatttt cccctcgaac actttttaag 9180
   ccgtagataa acaggctggg acacttcaca tgagcgaaaa atacatcgtc acctgggaca 9240
   tgttgcagat ccatgcacgt aaactcgcaa gccgactgat gccttctgaa caatggaaag 9300
   gcattattgc cgtaagccgt ggcggtctgt accgggtgcg ttactggcgc gtgaactggg 9360
   tattcgtcat gtcgataccg tttgtatttc cagctacgat cacgacaacc agcgcgagct 9420
   taaagtgctg aaacgcgcag aaggcgatgg cgaaggcttc atcgttattg atgacctggt 9480
   ggataccggt ggtactgcgg ttgcgattcg tgaaatgtat ccaaaagcgc actttgtcac 9540
   catcttcgca aaaccggctg gtcgtccgct ggttgatgac tatgttgttg atatcccgca 9600
   agatacctgg attgaacagc cgtgggatat gggcgtcgta ttcgtcccgc caatctccgg 9660
   tcgctaatct tttcaacgcc tggcactgcc gggcgttgtt ctttttaact tcaggcgggt 9720
   tacaatagtt tccagtaagt attctggagg ctgcatccat gacacaggca aacctgagcg 9780
   aaaccctgtt caaaccccgc tttaaacatc ctgaaacctc gacgctagtc cgccgcttta 9840
   atcacggcgc acaaccgcct gtgcagtcgg cccttgatgg taaaaccatc cctcactggt 9900
   atcgcatgat taaccgtctg atgtggatct ggcgcggcat tgacccacgc gaaatcctcg 9960
   acgtccaggc acgtattgtg atgagcgatg ccgaacgtac cgacgatgat ttatacgata 10020
   cggtgattgg ctaccgtggc ggcaactgga tttatgagtg ggccccggat ctttgtgaag 10080
   gaaccttact tctgtggtgt gacataattg gacaaactac ctacagagat ttaaagctct 10140
   aaggtaaata taaaattttt aagtgtataa tgtgttaaac tactgattct aattgtttgt 10200
   gtattttaga ttccaaccta tggaactgat gaatgggagc agtggtggaa tgcctttaat 10260
   gaggaaaacc tgttttgctc agaagaaatg ccatctagtg atgatgaggc tactgctgac 10320
   tctcaacatt ctactcctcc aaaaaagaag agaaaggtag aagaccccaa ggactttcct    10380
   tcagaattgc taagtttttt gagtcatgct gtgtttagta atagaactct tgcttgcttt    10440
   gctatttaca ccacaaagga aaaagctgca ctgctataca agaaaattat ggaaaaatat    10500
   tctgtaacct ttataagtag gcataacagt tataatcata acatactgtt ttttcttact    10560
   ccacacaggc atagagtgtc tgctattaat aactatgctc aaaaattgtg tacctttagc    10620
   tttttaattt gtaaaggggt taataaggaa tatttgatgt atagtgcctt gactagagat    10680
   cataatcagc cataccacat ttgtagaggt tttacttgct ttaaaaaacc tcccacacct    10740
   ccccctgaac ctgaaacata aaatgaatgc aattgttgtt gttaacttgt ttattgcagc    10800
   ttataatggt tacaaataaa gcaatagcat cacaaatttc acaaataaag catttttttc    10860
   actgcattct agttgtggtt tgtccaaact catcaatgta tcttatcatg tctggatcaa    10920
   ctggataact caagctaacc aaaatcatcc caaacttccc accccatacc ctattaccac    10980
   tgccaattac ctagtggttt catttactct aaacctgtga ttcctctgaa ttattttcat    11040
   tttaaagaaa ttgtatttgt taaatatgta ctacaaactt agtag                    11085
<210> 9
   <211> 51
   <212> RNA
   <213> Artificial Sequence
<220>
   <223> mouse shCIITA
<400> 9
   gctgacctcc cgtgtaaatg attcaagaga tcatttacac gggaggtcag c 51
<210> 10
   <211> 20
   <212> DNA
   <213> Artificial Sequence
<220>
   <223> shRNA for silencing HLA-DRA, HLA-DPA1 and HLA-DQA
<400> 10
   gactgcaggg tggagcactg 20
<210> 11
   <211> 21
   <212> DNA
   <213> Artificial Sequence
<220>
   <223> shRNA for silencing HLA-DRA, HLA-DPA1 and HLA-DQA
<400> 11
   tatgactgca aggtggagca c 21
<210> 12
   <211> 21
   <212> DNA
   <213> Artificial Sequence
<220>
   <223> shCIITA
<400> 12
   aagagaccag ggaggcttat g 21
<210> 13
   <211> 264
   <212> DNA
   <213> Artificial Sequence
<220>
   <223> U6 promoter
<400> 13
   aaggtcgggc aggaagaggg cctatttccc atgattcctt catatttgca tatacgatac     60
   aaggctgtta gagagataat tagaattaat ttgactgtaa acacaaagat attagtacaa    120
   aatacgtgac gtagaaagta ataatttctt gggtagtttg cagttttaaa attatgtttt    180
   aaaatggact atcatatgct taccgtaact tgaaagtatt tcgatttctt ggctttatat    240
   atcttgtgga aaggacgaaa cacc                                           264